# EUROPEAN PATENT SPECIFICATION

(11) **EP 1 667 748 B1**
(45) Date of publication and mention of the grant of the patent: **25.05.2011**
(21) Application number: 04770064.6
(22) Date of filing: 23.09.2004
(51) Int. Cl.: A61L 31/02, A61L 31/14

(54) **BIODEGRADABLE AND/OR BIOABSORBABLE MEMBER FOR VASCULAR SEALING**
BIOLOGISCH ABBAUBARES UND/ODER BIOABSORBIERBARES GLIED FÜR DEN GEFÄSSVERSCHLUSS
ELEMENT BIODEGRADABLE ET/OU BIOABSORBABLE POUR L'OBTURATION VASCULAIRE

(30) Priority: 28.09.2003 WO PCT/IB03/04289
(43) Date of publication of application: 14.06.2006
(73) Proprietor: Schnyder, Guido, 1801 Le Mont-Pélerin (CH); Rouvinez, Gilles, 1807 Blonay (CH)
(72) Inventor: Schnyder, Guido, 1801 Le Mont-Pélerin (CH); Rouvinez, Gilles, 1807 Blonay (CH)
(86) International application number: PCT/IB2004/051833
(87) International publication number: WO 2005/030285

(56) References cited:
- WO-A1-02/053202
- US-A- 4 602 632
- US-A1- 2002 004 060
- US-B1- 6 287 332

## Description

### Field of the invention

The present invention relates to closing apertures in body tissues caused by punctures. More specifically, the present invention relates to sealing members such as a staple, snap or rivet for scarring such apertures.

### Background

It frequently happens that portions of internal body tissue need to be sealed together. Often this need is a result of a cardiac or peripheral vascular catheterization. The art of sealing body tissues will therefore be discussed with a particular emphasis on closing apertures resulting of such interventions. Cardiac or peripheral vascular catheterizations are well known procedures that typically involve the making of a puncture in the femoral, radial or brachial artery to allow catheter insertions for diagnosis or treatment of cardiovascular or peripheral vascular diseases. After diagnostic and/or interventional catheterizations, the puncture formed by the insertion of the catheter must be closed following removal of the catheter. The puncture opening in the artery has a typical size in the range of 4-6 French for diagnostic procedures and in the range of 6-15 French for interventional procedures. Traditionally, manual or mechanical compressions are applied to puncture sites for at least 20 minutes and up to 6 hours after removal of the catheter. Other traditional methods for sealing the puncture site include the use of thrombotic or collagen plugs, patches, or other suturing methods.

In particular, patients who have had a femoral artery puncture should remain at strict bed rest, sometimes with a heavy sandbag on their groin for several hours, to ensure adequate hemostasis.

Traditional methods of hemostasis, as described above, following a femoral artery access have many pitfalls. Patients have to remain on their back for many hours having their leg with the access site stretched, which is felt by many patients as a great discomfort, often greater than the entire interventional procedure. Furthermore, the weight of a sandbag on the femoral artery often causes the lower leg to tingle or go numb. In addition, the longer it takes to obtain secure sealing of the wound (up to 24 hours), the higher the risk of local complications such as hematoma, false aneurysms, local or systemic infections and/or acute vessel occlusions. This makes wound site management the longer critical care item involving additional costs, greater patient discomfort, and increased risk of complications.

Surgical stapling instruments have been proposed to solve some of the aforementioned problems associated with vascular procedures. U.S. Pat. No. 5,709,335 (Heck) discloses a wholly distal surgical stapler for attaching a tubular vessel having two untethered ends. This stapler is especially useful for making the primary permanent anastomotic connection of a bypass vein to a coronary artery or to the aorta. This stapler needs to be temporarily placed within the tubular vessel (e.g., vein or artery). Such staplers are useful for stapling a graft vein or the like. However, they are inappropriate when the entirety of the tubular tissue is not accessible, such as during vascular sealing following cardiac or peripheral vascular catheterizations.

Another example of a surgical stapling instruments is found in U.S. Pat. No. 5,695,504 (Gifford et al.), which discloses a stapler to perform end-to-side anastomosis between a graft vessel and the wall of a target vessel. The end of a graft vessel has to be passed through an inner sleeve of the stapler until the end of the vessel extends from the distal end of the stapler. The distal end of the graft is then permanently stapled to the wall of the target vessel. Such staplers are useful for attaching two tubular tissues together. However, they are inadequate for sealing vascular punctures, such as those created to perform cardiac or peripheral vascular catheterizations.

In general, staples used during cardiovascular surgery, and in particular staples of the type disclosed in U.S. Pat. No. 5,709,335 and 5,695,504, are known to be made out of durable materials such as for example titanium or stainless steel, which will outlive patients.

U.S. Pat. No. 6,506,210 (assigned to AngioLink Corporation) discloses a wound site management and wound closure system involving a slightly different stapler. The staple does not require intraluminal delivery and is appropriate for sealing vascular punctures, such as those created to perform cardiac or peripheral vascular catheterizations. The assignee's system (EVS™) involves a staple made of titanium, a biocompatible material with appropriate mechanical properties to allow efficient sealing of the puncture site. The assignee has emphasized the importance of a permanent durable material, such as titanium, permitting X-ray puncture localization for subsequent interventions (Transcatheter Cardiovascular Therapeutics, Washington: September, 2002).

The use of inert durable materials such as titanium, stainless steal or ceramic, which do not dissolve during the lifetime of the patient, results in the implant of a permanent foreign body. This permanent implant may create a chronic irritation of the tissue surrounding the staple.

Another drawback is the potential damaging effect of an external compression that can trap the underlying vessel between such a rigid permanent staple and the head of the femur. A laceration of the underlying vessel could therefore be caused by shocks, for example during falls which are common in the case of the many older debilitated patients undergoing cardiac or peripheral catheterizations.

Puncture of the femoral artery is ideally performed at the level of the common femoral artery, because of its relatively large size and compressability. The latter depends on its course over bone, against which it can be readily compressed to achieve hemostasis. If the puncture is too proximal, the external iliac artery may be entered, increasing the risk of retroperitoneal hemorrhage; if the puncture is too distal, either the profunda femoral artery or the superficial femoral artery can be punctured, with a risk of local complications such as vessel laceration, pseudoaneurysm, arteriovenous fistula, thrombosis, or excessive bleeding.

The anatomy and in particular the length of the common femoral artery have been quantified to allow optimal puncture thereof. It has been found that the ideal puncture site is located in the area overlapping the upper inner quadrant of the femoral head, as it accurately predicts access in the common femoral artery whose length ranges from 0 to 11cm (mean: 6.7cm). This limits the remaining accessible segment of the common femoral artery to a length of about 2.0cm (Schnyder, G. et al., in Catheterization and Cardiovascular Interventions, vol. 53, pp. 289-295 [2001]). Since the staple described in U.S. Pat. No. 6,506,210 has a diameter of 3-4mm when fully expanded during implantation, it follows that such a staple can only be used a limited number of times (two to three) at the same location. This is problematic for treating patients with extensive vascular disease, who require a plurality of interventions.

### Summary of the invention

It is an object of the present invention to reduce the risk of injuries or other complications following application of the above described prior art sealing members. The present invention relates to a member for urging together two or more portions of body tissue that form a wound caused by a puncture, in particular a puncture resulting from a catheter-based intervention, and maintaining these portions together until they are secured together by scarring thereof. According to the invention, the member is made of a material selected from at least one of metals, alloys and ceramic compounds thereof such as oxides, which material is bioresorbable and/or biodegradable.

The above portions of body tissue may forms a wound, such as a puncture resulting from a catheter-based intervention. However, in the context of the present invention, any puncture is contemplated, accidental or intentional.

The sealing member of the present invention can be used in and around the femoral, radial, and brachial arteries after coronary, cardiac or peripheral vascular procedures. The sealing member can be a staple, snap or rivet.

A bioresorbable material is a material that is transformed, when present in a body tissue, into smaller elements - such as colloidal particles - with the newly formed elements remaining in the body as traceable elements containing for example titanium, zirconium, niobium oxide, tantalum, silicon and lithium or compounds thereof.

A biodegradable material is a material that is transformed, when present in body tissue, into smaller elements - such as soluble salts - with the newly formed elements either remaining in the surrounding tissue as fine undetectable precipitates or dissolving and being ultimately eliminated from the body. These elements include for example magnesium, zinc, sodium, potassium, calcium, iron and manganese salts or compounds thereof.

The nature of the materials used in the members of the present invention - bioresorbable / biodegradable - are opposed to the prior art biocompatible or bioabsorbable members, which both permanently remain in the local body tissue without undergoing any major structural changes. The biocompatible materials remain inert and do not trigger any tissue counter-reaction, while the bioabsorbable materials are ultimately incorporated permanently into the surrounding tissue.

The sealing member of the present invention may be used to close an artery or vein following a diagnostic or interventional procedure. More generally, the member may be used for any tissue repair.

As opposed to prior art permanent staples, the sealing member according to the inventions is present in the human body for a limited period of time sufficient to secure scarring, thereby tremendously reducing the risk of subsequent vessel injuries by external compression and permitting unlimited repetitive use for future interventions.

The implantable, bioresorbable and/or biodegradable sealing member may comprise a combination of materials which dissolve in the human body without any harmful effects on the person that wears the member. The materials of a sealing member can be a combination of metals, polymers or mixture thereof or any other substances such that degradation products originating from the sealing member in the form of particles of at least one of soluble salts, fine particles (e.g. 0.1µm-50µm) and/or colloidal particles (e.g. 5nm-0.1µm).

The sealing member is advantageously made of materials which can undergo adequate plastic deformation (to enable insertion of the member with negligible elastic recoil) and strong mechanical properties so as to secure the wound site despite shear forces generated by blood flow within the vessel and by the surrounding tissue when the patient is moving (i.e. walking, climbing stairs, etc...).

It will be appreciated throughout the following description that the members of the present invention are made of any bioresorbable and/or biodegradable material that fulfills the above required properties of deformability and mechanical resistance.

Such materials have previously been used to manufacture vessel wall supports or stents, as described in U.S. Pat. No. 6,287,332 (assigned to Biotronik Mess- und Therapiegeraete GmbH & Co.). Such stents are used to minimize inflammatory reaction so as to reduce the production of scar material upon implantation, whereby instent restenosis or renarrowing of the previously treated vessel segment by scar material is prevented. Surprisingly, sealing members made of these bioresorbable and/or biodegradable materials according to the invention do not prevent secure scarring but permit adequate healing.

The material of the member can be made of a combination of metals which can dissolve in the body without significantly forming bio-incompatible decomposition products. The material may dissolve at a rate in the range from 0.1 to 5 mg/day, in particular from 0.5 to 2mg/day. A sealing member made of this material may be entirely dissolved within 1 to 300 days, in particular 5 to 100 days, such as 10 to 50 days.

Such a temporary sealing member combines the mechanical properties of metals with the bioresorbability of polymer-based materials. Immediate mechanical sealing of an access site can be achieved with this non-permanent sealing member. The sealing member may be entirely dissolved after complete scarring of the access site or after sufficient scar material has been formed to permit complete sealing even upon dissolution or resorption of the sealing member. The sealing member should retain it's mechanical properties to maintain the body tissues urged together for at least 1 day, in particular for at least 3 days.

In a first embodiment of the invention, the sealing member is made of a metal alloy suitable for biocompatible decomposition, as explained in detail below. Consequently, in this embodiment, the metal alloy consists essentially of a combination of materials that will decompose in the body comparatively rapidly - within a period of days or weeks or months, but preferably no more than 12 months - forming harmless products.

To obtain a substantially uniform decomposition, such an alloy may comprise a component A which covers itself with a protective oxide coat. This component A can be selected from one or several metals of the group consisting of magnesium, titanium, zirconium, niobium, tantalum, zinc or silicon. Moreover, to obtain substantially uniform dissolution of this oxide coat, a component B, that possesses sufficient solubility in interstitial fluids or blood - such as lithium, sodium, potassium, calcium, iron or manganese - can be added to the alloy.

Suitable metals for the alloy include metals that are naturally present in the human body (magnesium, zinc, sodium, potassium, calcium, iron and manganese) or that are nontoxic (titanium, zirconium, niobium, tantalum, silicon and lithium). The combination of a passivating and a soluble component ensures a timely and uniform decomposition into biocompatible breakdown products. The decomposition rate can be set by the ratio of the two components.

The alloy can be such that the decomposition products are soluble salts, in particular sodium, potassium, calcium, iron or zinc salts, or non-soluble decomposition products, such as titanium, tantalum or niobium oxide, in the form of colloidal particles. The decomposition rate is advantageously adjusted by the composition so that gases, when formed, dissolve physically without forming any macroscopic gas bubbles. For example, hydrogen gas evolves during the decomposition of lithium, sodium, potassium, magnesium, calcium or zinc salts.

For instance, an alloy of lithium and magnesium, can be used as a possible alloy which is however optimized with a view to increase fatigue durability for the field of application mentioned above. The weight ratio magnesium/lithium can be of the order of 60/40, fatigue durability being increased by the addition of further components, such as zinc, or by gassing by hydrogen. Also, special melting and forging methods can be used to increase the fatigue durability.

A sodium-magnesium alloy may be used to make the sealing member. Since sodium hydroxide as a decomposition product possesses a high solubility, this alloy can brake down without voluminous encrusting. Sodium dissolves and magnesium hydroxide forms a fine precipitate which may deposit without risk in the surrounding tissue.

Another decomposable combination of metal materials is a zinc-titanium alloy, with a percentage by weight of titanium in the range of 0.1% to 1%. This combination precludes the comparatively strong crystalline growth of zinc during use, which would cause a comparatively brittle and fragile behavior of the sealing member. The addition of titanium leads to the formation of a Zn₁₅Ti phase at the crystal boundaries, which precludes any further crystalline growth. This reduction of the grain size generally improves the ductility, in particular it increases the elongation at rupture.

If gold is added to this alloy at a percentage by weight of 0.1% to 2%, a further reduction of the grain size is achieved when the material cures. This further improves the tensile strength of the material.

In another embodiment of the invention, the stealing member comprises a support body and a local electrode for use as an electrochemical device. The support body can be made of a substantially pure metal. Usually, the local electrode is made of a second metal and is in contact with the support body. This local electrode can be a coat on the sealing member or is fixed onto the sealing support body by electroplating or by laser welding. The contact between the support body and the local electrode produces a contact voltage and a resulting current that leads to active degradation of the sealing member. The degradation rate and thus the decomposition rate of the sealing member can be controlled by the size of the local electrode and by the selection of the metals of the sealing member.

### Brief Description of the Drawings

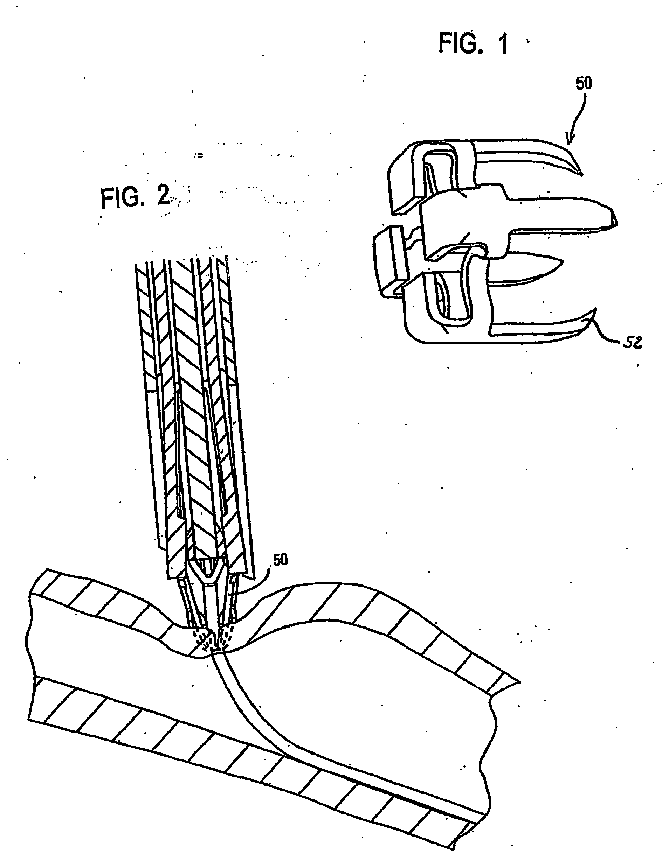

The appended Figures show comparative staples as disclosed in the abovementioned US 6,506,210, wherein: Fig. 1 shows a staple; and Fig. 2 shows a staple which is being deployed with the aid of a stapler into tissue.

### Detailed description

Fig. 1 shows a comparative staple 50 with a plurality of prongs 52 that can be deployed into tissue around a wound site to close a wound in a vessel wall caused by a puncture formed during catheterization. Fig. 2 shows staple 50 after having been deployed with the aid of a stapler into tissue to close a wound. During stapling, prongs 52 can be first extended outwardly so as to grasp large portions of tissue around the wound, and so that insertion of prongs 52 into the tissue occurs away from the wound, thereby providing a more consistent wound closure. Staples and process for inserting them into tissue around a wound is explained in greater detail in US 6,506,210.

This prior art staple 50 is made of biocompatible and/or bioabsorbable materials, including for example titanium, (and titanium alloys) stainless steel, polymeric materials (synthetic and/or natural), ceramic, etc. As opposed to the members, in particular staples, of the present invention, this prior art staple is not made of a bioresorbable and/or biodegradable material, in particular one of the suitable titanium alloys containing one of lithium, sodium, potassium, manganese calcium and iron, as explained above.

The invention will be further explained in the following Exemples:

### Example 1

A bioresorbable and/or biodegradable sealing member according to the invention can be made from an alloy containing zinc as the component A and calcium as the component B. The weight ratio that zinc bears to calcium amounts to 25/1. This Zn-Ca alloy forms soluble salts as degradation products, such as calcium hydroxide which possesses such a high solubility that the solubility product is not transgressed during slow decomposition over several weeks or months. This hydroxide is transported in dissolved form by interstitial fluids or blood and is metabolized.

To improve the mechanical properties of the sealing member, such as ductility, hardness and tensile strength, suitable alloy constituents can be added in low concentrations. For instance, phosphorus may be added to the alloy in an amount of the order of a few percents.

### Example 2

A bioresorbable and/or biodegradable metal sealing member acting as a local electrochemical device according to the invention can comprise a support body and a local electrode. The support body is made of substantially pure zinc which dissolves - as electroplating tests show - without production of gases and without the formation of oxide at currents of some milliamps. The local electrode is made of gold and is in contact with the zinc support body. This local gold electrode is fixed onto the sealing support body by electroplating or by laser welding. The contact between the zinc support body and the local gold electrode produces a contact voltage and a resulting current that leads to active degradation of the sealing member. The exchange current as a whole is determined by the size of the gold electrode. The degradation rate and thus the decomposition rate of the sealing member can be adjusted by the size of the local gold electrode.

Tests have shown that an exchange current arises between the sealing member's support body and the local electrode after few minutes and remains constant for several days in such a sealing member. Hence, a constant decomposition rate can be attained and a member of 10 mg will dissolve within approximately 30 to 40 days at a corrosion current of 10 µA.

### Example 3

A bioresorbable metal sealing member according to the invention can be made form a ZnTi alloy with a Ti weight percentage of 0.1% to 1%. In a further improved embodiment of this example, a precious metal in the form of gold can be added at a weight percentage of 0.1% to 2%, the Ti weight percentage remaining unchanged so that the member consists of a ZnAuTi alloy. These two alloys also exhibit a biocompatible decomposition behavior and are thus regarded as bioresorbable sealing members.

## Claims

1. A member, such as a staple or rivet, for urging together two or more portions of tissue of a body which tissue portions form a wound caused by a puncture, in particular a puncture resulting from a catheter-based intervention, and maintaining said portions together until said portions are secured together by scarring thereof, wherein said member is made of a material selected from at least one of metals, alloys and ceramic compounds thereof, such as oxides, said material being:
- a bioresorbable material which is transformable in said tissue into smaller elements, such as colloidal particles, that remain in said body as traceable elements; and/or
- a biodegradable material which is transformable in said tissue into smaller elements, such as soluble salts, that remain in surrounding tissue as fine undetectable precipitates or that dissolve and are ultimately eliminated from said body.

2. The member of claim 1, wherein said material is a metal alloy containing: a first component which covers itself with a protective oxide coat; and a second component which ensure sufficient dissolution of the oxide coat.

3. The member of claim 2, wherein the first component comprises at least one metal selected from magnesium, titanium, zirconium, niobium, tantalum, zinc and silicon and the second component comprises at least one metal selected from lithium, sodium, potassium, manganese calcium and iron.

4. The member of claim 2 or 3, wherein the components of the metal alloy are selected such that corrosion products originate therefrom in the form of soluble salts, fine particles or colloidal particles or a mixture thereof.

5. The member of claim 2, 3 or 4, wherein the alloy contains zinc as a corrosion-inhibiting component.

6. The member of claim 5, wherein the alloy contains zinc and calcium.

7. The member of claim 6, wherein the alloy has a zinc/calcium weight ratio of at least 21/1.

8. The member of claim 2, 3, or 4, wherein the alloy contains sodium and magnesium.

9. The member of claim 1, wherein the bioresorbable and/or biodegradable material is an alloy of zinc and titanium.

10. The member of claim 9, wherein the zinc-titanium alloy has a weight percentage of titanium of 0.1% to 1%.

11. The member of claim 10, wherein an amount of 0.1 to 2 weight% gold is added as a further component to the zinc titanium alloy.

12. The member of claim 1, wherein the bioresorbable and/or biodegradable sealing member comprises a support body made of a substantially pure first metal and a local electrode made of a second metal which is in contact with the support body to produce a contact voltage and a resulting current that leads to active degradation of the sealing member.

13. The member of claim 12, wherein the local electrode is a coat on the support body.

14. The member of claim 12, wherein the local electrode is a metal part attached to the support body.

15. The member of claim 12, 13 or 14 wherein the support body consists essentially of zinc.

16. The member of claim 12, 13 or 14 wherein the local electrode consists essentially of a precious metal.

17. The member of claim 13, wherein said coat is deposited by electroplating or spluttering.

18. The member of any preceding claim, wherein the sealing member is made of a phosphorus-containing alloy.

19. The member of any preceding claim, which is a hydrogen-treated alloy.

20. The member of any preceding claim, which is made of an alloy which during use corrodes at such a rate that gases arising during corrosion physically dissolves in a body fluid to which the alloy is exposed.

## Patentansprüche

1. Ein Glied, wie eine Klammer oder eine Niete, um eine Wunde die nach Punktion, im speziellen durch einen katheterbedingten Eingriff, entstanden ist, bestehend aus zwei oder mehr Gewebeteile eines Körpers zusammenzudrängen, um die Wundränder zusammenzuhalten bis zur Heilung und Narbenbildung, wobei das betreffende Glied aus einem Material gemacht ist, wobei das Material mindestens eines von Metalle, Legierungen, Keramiken und Mischungen beinhalted, z. B. Oxid, wobei das betreffende Material
- ein bioresorbierbares Material das im Gewebe in kleine Elemente zerfällt wie kolloidale Partikel, die im Körper nachweisbar bleiben; und/oder
- ein biodegradierbares Material, welches in kleine Elemente zerfällt, wie lösbare Salze, die im umliegenden Gewebe als kleinste nicht nachweisbare Präzipitate verbleiben oder die sich auflösen und nicht mehr nachweisbar sind ist.

2. Das Glied des Anspruchs 1, wobei das Material eine Metall-Legierung ist, die eine erste Komponente die sich mit einer Oxid Schutzschicht sich selbst bedeckt und eine zweite Komponente die eine genügende Auflösung der Oxid-Schicht garantiert.

3. Das Glied des Anspruchs 2, wobei die erste Komponent mindestens eines der folgenden Metalle beinhalted: Magnesium, Titan, Zirkonium, Niob, Tantal, Zink, Silizium, und wobei das Material der zweiten Komponente aus einem der folgenden Metalle: Lithium, Natrium, Kalium, Mangan, Kalzium und Eisen.

4. Das Glied des Anspruchs 2 und 3, wobei die Komponente der Metall-Legierung so gewählt sind, dass deren Korrosions-Produkte in lösliche Salze, in feine Partikel oder kolloidale Partikel oder eine Mischung davon zerfällt.

5. Das Glied des Anspruchs 2, 3 oder 4, wobei die Legierung Zink als korrosionshemmende Komponente beinhaltet.

6. Das Glied des Anspruchs 5, wobei die Legierung Zink und Kalzium als korrosionshemmende Komponente beinhaltet.

7. Das Glied des Anspruchs 6, wobei die Legierung einen Zink/Kalzium Quotienten von mindestens 21/1 hat.

8. Das Glied des Anspruchs 2, 3 oder 4, wobei die Legierung Natrium und Magnesium beinhaltet.

9. Das Glied des Anspruchs 1, wobei das bioresorbierbare oder biodegradierbare Material eine Legierung aus Zink und Titan ist.

10. Das Glied des Anspruchs 9, wobei die Zink-Titan-Legierung ein Titan Gewichtsprozent von 0.1% bis 1% hat.

11. Das Glied des Anspruchs 10, wobei die Zink-Titan-Legierung 0.1 bis 2 Gewichtsprozent Gold als zusätzliche Komponente hat.

12. Das Glied des Anspruchs 1, wobei das betreffende bioresorbierbare und/oder biodegradierbare Glied ein Stützkörper beinhaltet der ein substanziell reines Erstmetall und eine lokale Elektrode beinhalted, wobei die Elektrode aus einem zweiten Metall gemacht ist und in Kontakt mit dem Stützkörper steht um eine Kontaktspannung und ein daraus resultierender Strom zu produzieren, der die Degradierung des betreffenden Gliedes aktiviert.

13. Das Glied des Anspruchs 12, wobei die lokale Elektrode ein Mantel des Stützkörpers ist.

14. Das Glied des Anspruchs 12, wobei die lokale Elektrode ein an dem Stützkörper gebunden Metallstück ist.

15. Das Glied des Anspruchs 12, 13 oder 14, wobei der Stützkörper hauptsächlich aus Zink besteht.

16. Das Glied des Anspruchs 12, 13 oder 14, wobei der Stützkörper hauptsächlich aus Edelmetall besteht.

17. Das Glied des Anspruchs 13, wobei der Mantel durch Galvanotechnik oder Kathodenzerstäubung deponiert ist.

18. Das Glied irgend eines der vorgehenden Ansprüche, wobei das betreffende Glied aus einer Phosphor-enthaltenden Legierung gemacht ist.

19. Das Glied irgend eines der vorgehenden Ansprüche, wobei das betreffende Glied eine Hydrogen-behandelte Legierung ist.

20. Das Glied irgend eines der vorgehenden Ansprüche, das aus einer Legierung bestehen, die bei Benutzung derart rasch korrodieren, dass Gase bei der Korrosion entstehen, die sich in Körperflüssigkeiten auflösen in denen die Legierungen exponiert sind.

## Revendications

1. Un membre, tel une agrafe ou un rivet, pour réunir deux ou plusieurs parties de tissu d'un corps lesquelles forment une plaie occasionnée par une ponction, en particulier une ponction provenant d'une intervention par cathéter, et pour maintenir ensemble les dites parties de tissu jusqu'à ce que les dites parties soient maintenues ensemble par cicatrisation, ledit membre étant formé par au moins un matériau sélectionné parmi des métaux, des alliages, des céramiques et des combinaisons de ces-derniers, telles des oxydes, ledit matériau étant :
- un matériau biorésorbable qui se transforme dans le dit tissu en éléments plus petits, comme des particules colloïdales, qui restent dans le dit corps comme des éléments traçables ; et/ou
- un matériau biodégradable qui se transforme dans le dit tissu en éléments plus petits, comme des sels, qui restent dans les abords du tissu sous forme de fins précipités non détectables ou qui se dissolvent et finalement sont éliminées du dit corps.

2. Le membre de la revendication 1, **caractérisé en ce que** le matériau est un alliage métallique comportant : un premier constituant qui se recouvre lui-même d'une revêtement protecteur d'oxyde ; et un deuxième constituant qui assure la dissolution suffisante de la pellicule d'oxyde.

3. Le membre de la revendication 2, dans lequel le premier constituant est formé d'au moins d'un métal sélectionné du magnésium, titane, zirconium, niobium, tantale, zinc et de silicium, et le deuxième constituant est formé d'au moins d'un métal sélectionné du lithium, sodium, potassium, manganèse, calcium et fer.

4. Le membre de la revendication 2 ou 3, dans lequel les composants de l'alliage métallique sont sélectionnés de telle façon à générer des produits de corrosion sous forme de sels solubles, de fines particules ou de particules colloïdales ou encore d'une combinaison de ces produits de corrosion.

5. Le membre de la revendication 2, 3 ou 4, dans lequel l'alliage contient du zinc comme élément inhibant la corrosion.

6. Le membre de la revendication 5, dans lequel l'alliage contient du zinc et du calcium.

7. Le membre de la revendication 6, dans lequel l'alliage zinc/calcium a un rapport poids d'au moins 21/1.

8. Le membre de la revendication 2,3, ou 4, dans lequel l'alliage contient du sodium et du manganèse.

9. Le membre de la revendication 1, dans lequel le matériau biorésorbable et/ou biodégradable est un alliage de zinc et de titane.

10. Le membre de la revendication 9, dans lequel l'alliage zinc/titane a un pourcentage de poids de titane allant de 0.1 % à 1.0%.

11. Le membre de la revendication 10, dans lequel une quantité d'or allant d'un pourcentage poids de 0.1% à 2% est ajoutée comme constituant supplémentaire à l'alliage de zinc et de titane.

12. Le membre de la revendication 1, dans lequel le système de fermeture biorésorbable et/ou biodégradable est formé d'une part par d'un corps de soutien formé d'un premier métal sensiblement pur et d'autre part d'une électrode locale formée d'un deuxième métal en contact avec celui-ci et provoquant une tension de contact générant un courant provoquant une dégradation active du système de fermeture.

13. Le membre de la revendication 12, dans lequel l'électrode locale se présente sous forme d'un revêtement du corps de soutien.

14. Le membre de la revendication 12, dans lequel l'électrode locale se présente sous forme d'une pièce métallique fixée au corps de soutien.

15. Le membre de la revendication 12, 13 ou 14 dans lequel le corps de soutien est essentiellement formé de zinc.

16. Le membre de la revendication 12, 13 ou 14 dans lequel le corps de soutien est essentiellement formé d'un métal précieux.

17. Le membre de la revendication 13, dans lequel le dit revêtement est déposé par galvanoplastie ou pulvérisation.

18. Le membre de l'une quelconque des revendications précédentes, dans lequel le système de fermeture est formé d'un alliage contenant du phosphore.

19. Le membre de l'une quelconque des revendications précédentes, qui est un alliage traité à l'hydrogène.

20. Le membre de l'une quelconque des revendications précédentes, qui est formé d'un alliage qui à l'emploi se corrode à une vitesse telle que les gaz formés durant le processus de corrosion se dissolvent physiquement dans le liquide auquel l'alliage est exposé.
